(19) Europäisches Patentamt / European Patent Office / Office européen des brevets

(11) **EP 3 270 973 B1**

(12) **EUROPEAN PATENT SPECIFICATION**

(45) Date of publication and mention
of the grant of the patent:
**09.01.2019 Bulletin 2019/02**

(51) Int Cl.:
*A61K 47/38* (2006.01)       *A61K 9/14* (2006.01)
*A61K 9/48* (2006.01)       *A61K 9/28* (2006.01)

(21) Application number: **16714625.7**

(22) Date of filing: **08.03.2016**

(86) International application number:
**PCT/US2016/021313**

(87) International publication number:
**WO 2016/148970 (22.09.2016 Gazette 2016/38)**

(54) **AQUEOUS SOLUTION OF AN ESTERIFIED CELLULOSE ETHER**

WÄSSRIGE LÖSUNG EINES VERESTERTEN CELLULOSEETHERS

SOLUTION AQUEUSE D'UN ÉTHER DE CELLULOSE ESTÉRIFIÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.03.2015 US 201562133511 P**

(43) Date of publication of application:
**24.01.2018 Bulletin 2018/04**

(73) Proprietor: **Dow Global Technologies LLC
Midland, MI 48674 (US)**

(72) Inventors:
• **PETERMANN, Oliver
29699 Bomlitz (DE)**
• **KNARR, Matthias
29699 Bomlitz (DE)**

(74) Representative: **f & e patent
Braunsberger Feld 29
51429 Bergisch Gladbach (DE)**

(56) References cited:
**WO-A1-2014/137777**

**Description**

FIELD

This invention concerns an aqueous composition comprising an esterified cellulose ether, a process for producing it and its use.

INTRODUCTION

Esters of cellulose ethers, their uses and processes for preparing them are generally known in the art, for example for improving the water solubility of poorly or moderately water-soluble drugs or for preparing capsules or coatings. U.S. Patent No. 4,226,981 discloses a process for preparing mixed esters of cellulose ethers, such as HPMCAS.

International Patent Application WO 2005/115330 discloses hydroxypropyl methyl cellulose acetate succinate (HPMCAS) polymers with a specific combination of degrees of substitution. The HPMCAS polymer has a degree of substitution of succinoyl groups (DOSs) of at least 0.02, a degree of substitution of acetyl groups ($DOS_{Ac}$) of at least 0.65 and a sum of $DOS_{Ac}$ and $DOS_S$ of at least 0.85. WO 2005/115330 discloses that the increased acetate substitution allows increased solubility of active agents in spray-dried solutions, while the increased succinate substitution increases the solubility of the polymer in aqueous solution.

When the esterified cellulose ethers comprise ester groups which carry carboxylic groups, such as HPMCAS, the solubility of the esterified cellulose ethers in aqueous liquids is typically dependent on the pH. The solubility of HPMCAS in aqueous liquids is pH-dependent due to the presence of succinate groups, also called succinyl groups or succinoyl groups. HPMCAS is known as enteric polymer for pharmaceutical dosage forms. In the acidic environment of the stomach HPMCAS is protonated and therefore insoluble. HPMCAS undergoes deprotonation and becomes soluble in the small intestine, which is an environment of higher pH. The pH-dependent solubility is dependent on the degree of substitution of acidic functional groups. The dissolution time of various types of HPMCAS dependent on pH and on the degree of neutralization of HPMCAS is discussed in detail in McGinity, James W. Aqueous Polymeric Coatings for Pharmaceutical Dosage Forms, New York: M. Dekker, 1989, pages 105 - 113. This publication illustrates in Fig. 16 on p. 112 the dissolution time of several grades of HPMCAS, which have different degrees of substitution with succinoyl, acetyl and methoxyl groups, in pure water and in 0.1 N NaCl depending on the degree of neutralization of the HPMCAS. Depending on the HPMCAS and the presence or absence of NaCl, HPMCAS is soluble when it has a degree of neutralization between about 0.55 and 1. Below a degree of neutralization of about 0.55, all HPMCAS grades are insoluble in pure water and in 0.1 N NaCl.

Dosage forms coated with esterified cellulose ethers such as HPMCAS protect the drug from inactivation or degradation in the acidic environment of the stomach or prevent irritation of the stomach by the drug but release the drug in the small intestine. US Patent No. 4,365,060 discloses enterosoluble capsules.

International Patent Application WO 2013/164121 teaches that many techniques for preparing capsules still require the combination of an enteric (acid insoluble) polymer and a conventional non-enteric polymer, require salts or pH regulators leading to water sensitivity or brittleness of the resulting capsule shells, require multiple processing steps, and/or need to be processed in non-aqueous media. To solve these problems, WO 2013/164121 discloses an aqueous composition comprising HPMCAS polymer dispersed in water, wherein the polymer is partially neutralized with at least one alkaline material, such as ammonia, sodium hydroxide, calcium hydroxide, potassium hydroxide, cationic polymers, and mixtures thereof. Unfortunately, the partial neutralization may impact the enteric properties of the capsules. E.g., stomach liquid may diffuse into the capsule upon ingestion when the capsule comprises partially neutralized HPMCAS.

In view of the great usefulness of esterified cellulose ethers comprising ester groups which carry carboxylic groups, such as HPMCAS, for improving the water solubility of poorly or moderately water-soluble drugs or for preparing capsules or coatings, there is the urgent need to find a way for dissolving such esterified cellulose ethers in aqueous liquids even when the carboxylic groups have a low degree of neutralization.

SUMMARY

Surprisingly, an efficient and simple process for least partially dissolving esterified cellulose ethers in an aqueous liquid has been found.

One aspect of the present invention is a process for producing an aqueous composition comprising at least 1 weight percent of an esterified cellulose ether dissolved in an aqueous liquid, wherein the esterified cellulose ether comprises groups of the formula - C(O) - R - COOH, wherein R is a divalent hydrocarbon group and the process comprises the step of

a) mixing the esterified cellulose ether with the aqueous liquid, and

b) maintaining or adjusting the degree of neutralization of the groups - C(O) - R - COOH of the esterified cellulose ether at or to less than 0.45 and setting the temperature of the mixture of the esterified cellulose ether and the aqueous liquid to less than 10 °C to at least partially dissolve the esterified cellulose ether in the aqueous liquid to provide the aqueous composition comprising at least 1 weight percent esterified cellulose ether dissolved in the aqueous liquid.

Another aspect of the present invention is a process for manufacturing capsule shells which comprises the steps of producing an aqueous composition comprising an esterified cellulose ether according to the above-mentioned process and contacting dipping pins having a higher temperature than the aqueous composition with the aqueous composition or with the portion of the aqueous composition wherein esterified cellulose ether is dissolved.

Yet another aspect of the present invention is a process for coating dosage forms which comprises the steps of producing an aqueous composition comprising an esterified cellulose ether according to the above-mentioned process and contacting dosage forms with the aqueous composition or with the portion of the aqueous composition wherein esterified cellulose ether is dissolved.

Yet another aspect of the present invention is a process for preparing a solid dispersion of an active ingredient in an esterified cellulose ether which comprises the steps of producing an aqueous composition comprising an esterified cellulose ether according to the above-mentioned process and dissolving an active ingredient in the aqueous composition or in the portion of the aqueous composition wherein esterified cellulose ether is dissolved, and drying the aqueous composition or the portion of the aqueous composition wherein esterified cellulose ether and active ingredient are dissolved to produce the solid dispersion of an active ingredient in an esterified cellulose ether.

Yet another aspect of the present invention is an aqueous composition comprising at least 1 weight percent of an esterified cellulose ether dissolved in an aqueous liquid, wherein i) the esterified cellulose ether comprises groups of the formula - C(O) - R - COOH having a degree of neutralization of less than 0.45, wherein R is a divalent hydrocarbon group, and ii) the aqueous composition has a temperature of no more than 10 °C.

BRIEF DESCRIPTION OF DRAWINGS

Fig. 1A, 2A and 3A are photographical representations of non-dissolved pieces of capsule shells in 0.1 N HCl.
Fig. 1B, 2B and 3B are photographical representations of aqueous buffer solutions of pH 6.8 into which the non-dissolved pieces of capsule shells shown in Fig. 1A, 2A and 3A have been placed; all pieces of capsule shells are dissolved in the aqueous buffer solutions of pH 6.8.

DESCRIPTION OF EMBODIMENTS

Surprisingly, it has been found that an esterified cellulose ether comprising groups of the formula - C(O) - R - COOH is at least partially dissolved in an aqueous liquid when a) the esterified cellulose ether is mixed with the aqueous liquid as defined further below and b) the degree of neutralization of the groups - C(O) - R - COOH of the esterified cellulose ether is maintained at or adjusted to less than 0.45 and the temperature of the mixture of the esterified cellulose ether and the aqueous liquid is set to less than 10 °C, preferably to less than 8 °C, more to preferably less than 5 °C, and particularly to 3 °C or less. When the temperature of the mixture has a temperature of 10 °C or more, such partial dissolution is not observed. Particularly at room temperature known esterified cellulose ethers comprising groups of the formula - C(O) - R - COOH do not dissolve in water to a noticeable degree when the degree of neutralization of the groups - C(O) - R - COOH of the esterified cellulose ether is less than 0.45.

The esterified cellulose ether used in the process of the present invention has a cellulose backbone having β-1,4 glycosidically bound D-glucopyranose repeating units, designated as anhydroglucose units in the context of this invention. The esterified cellulose ether preferably is an esterified alkyl cellulose, hydroxyalkyl cellulose or hydroxyalkyl alkylcellulose. This means that in the esterified cellulose ether at least a part of the hydroxyl groups of the anhydroglucose units are substituted by alkoxyl groups or hydroxyalkoxyl groups or a combination of alkoxyl and hydroxyalkoxyl groups. The hydroxyalkoxyl groups are typically hydroxymethoxyl, hydroxyethoxyl and/or hydroxypropoxyl groups. Hydroxyethoxyl and/or hydroxypropoxyl groups are preferred. Typically one or two kinds of hydroxyalkoxyl groups are present in the esterified cellulose ether. Preferably a single kind of hydroxyalkoxyl group, more preferably hydroxypropoxyl, is present. The alkoxyl groups are typically methoxyl, ethoxyl and/or propoxyl groups. Methoxyl groups are preferred. Illustrative of the above-defined esterified cellulose ethers are esterified alkylcelluloses, such as esterified methylcelluloses, ethylcelluloses, and propylcelluloses; esterified hydroxyalkylcelluloses, such as esterified hydroxyethylcelluloses, hydroxypropylcelluloses, and hydroxybutylcelluloses; and esterified hydroxyalkyl alkylcelluloses, such as esterified hydroxyethyl methylcelluloses, hydroxymethyl ethylcelluloses, ethyl hydroxyethylcelluloses, hydroxypropyl methylcelluloses, hydroxypropyl ethylcelluloses, hydroxybutyl methylcelluloses, and hydroxybutyl ethylcelluloses; and those having two or more hydroxyalkyl groups, such as esterified hydroxyethylhydroxypropyl methylcelluloses. Most preferably, the esterified cellulose ether is an esterified hydroxyalkyl methylcellulose, such as an esterified hydroxypropyl methylcellulose.

The degree of the substitution of hydroxyl groups of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the esterified cellulose ether. It is to be understood that during the hydroxyalkylation reaction the hydroxyl group of a hydroxyalkoxyl group bound to the cellulose backbone can be further etherified by an alkylation agent, e.g. a methylation agent, and/or a hydroxyalkylation agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone.

The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxy units are covalently bound to each other by ether bonding. Within this definition it is not important whether the terminal hydroxyl group of a hydroxyalkoxyl substituent is further alkylated, e.g. methylated, or not; both alkylated and non-alkylated hydroxyalkoxyl substituents are included for the determination of MS(hydroxyalkoxyl). The esterified cellulose ether generally has a molar substitution of hydroxyalkoxyl groups in the range 0.05 to 1.00, preferably 0.08 to 0.70, more preferably 0.15 to 0.60, most preferably 0.15 to 0.40, and particularly 0.20 to 0.40.

The average number of hydroxyl groups substituted by alkoxyl groups, such as methoxyl groups, per anhydroglucose unit, is designated as the degree of substitution of alkoxyl groups, DS(alkoxyl). In the above-given definition of DS, the term "hydroxyl groups substituted by alkoxyl groups" is to be construed within the present invention to include not only alkylated hydroxyl groups directly bound to the carbon atoms of the cellulose backbone, but also alkylated hydroxyl groups of hydroxyalkoxyl substituents bound to the cellulose backbone. The esterified cellulose ethers generally have a DS(alkoxyl) in the range of 1.0 to 2.5, preferably from 1.2 to 2.2, more preferably from 1.6 to 2.05, and most preferably from 1.7 to 2.05.

Most preferably the esterified cellulose ether is an esterified hydroxypropyl methylcellulose having a DS(methoxyl) within the ranges indicated above for DS(alkoxyl) and an MS(hydroxypropoxyl) within the ranges indicated above for MS(hydroxyalkoxyl).

The esterified cellulose ether comprises groups of the formula - C(O) - R - COOH, wherein R is a divalent hydrocarbon group, such as -C(O)-CH$_2$-CH$_2$-COOH, and optionally aliphatic monovalent acyl groups, such as acetyl, propionyl, or butyryl, such as n-butyryl or i-butyryl. Specific examples of esterified cellulose ethers are hydroxypropyl methylcellulose acetate succinate (HPMCAS), hydroxypropyl cellulose acetate succinate (HPCAS), hydroxybutyl methyl cellulose propionate succinate (HBMCPrS), hydroxyethyl hydroxypropyl cellulose propionate succinate (HEHPCPrS), or methyl cellulose acetate succinate (MCAS). Hydroxypropyl methylcellulose acetate succinate (HPMCAS) is the most preferred esterified cellulose ether.

The esterified cellulose ether generally has a degree of substitution of groups of formula -C(O) - R - COOH, such as succinoyl, of at least 0.01, preferably at least 0.05, and most preferably at least 0.10. The esterified cellulose ether generally has a degree of substitution of groups of formula -C(O) - R - COOH of up to 0.90, preferably up to 0.80, and more preferably up to 0.50. The esterified cellulose ethers generally have a degree of substitution of aliphatic monovalent acyl groups, such as acetyl, propionyl, or butyryl groups, of 0 or at least 0.05, preferably at least 0.10, and more preferably at least 0.25. The esterified cellulose ethers generally have a degree of substitution of aliphatic monovalent acyl groups of up to 0.95, preferably up to 0.80, and more preferably up to 0.70. The total degree of ester substitution is generally at least 0.05, preferably at least 0.10, and more preferably at least 0.20. The total degree of ester substitution is generally not more than 1.0, preferably not more than 0.90, and more preferably not more than 0.80.

The content of the acetate and succinate ester groups is determined according to "Hypromellose Acetate Succinate, United States Pharmacopia and National Formulary, NF 29, pp. 1548-1550". Reported values are corrected for volatiles (determined as described in section "loss on drying" in the above HPMCAS monograph). The method may be used in analogue manner to determine the content of propionyl, butyryl and other ester groups.

The content of ether groups in the esterified cellulose ether is determined in the same manner as described for "Hypromellose", United States Pharmacopeia and National Formulary, USP 35, pp 3467-3469.

The contents of ether and ester groups obtained by the above analyses are converted to DS and MS values of individual substituents according to the formulas below. The formulas may be used in analogue manner to determine the DS and MS of substituents of other cellulose ether esters.

% cellulose backbone

$$= 100 - \left( \%MeO * \frac{M(OCH_3) - M(OH)}{M(OCH_3)} \right)$$
$$- \left( \%HPO * \frac{M(OCH_2CH(OH)CH_3) - M(OH)}{M(OCH_2CH(OH)CH_3)} \right)$$
$$- \left( \%Acetyl * \frac{M(COCH_3) - M(H)}{M(COCH_3)} \right)$$
$$- \left( \%Succinoyl * \frac{M(COC_2H_4COOH) - M(H)}{M(COC_2H_4COOH)} \right)$$

$$DS(Me) = \frac{\dfrac{\%MeO}{M(OCH_3)}}{\dfrac{\%cellulose\ backbone}{M(AGU)}} \qquad MS(HP) = \frac{\dfrac{\%HPO}{M(HPO)}}{\dfrac{\%cellulose\ backbone}{M(AGU)}}$$

$$DS(Acetyl) = \frac{\dfrac{\%Acetyl}{M(Acetyl)}}{\dfrac{\%cellulose\ backbone}{M(AGU)}} \qquad DS(Succinoyl) = \frac{\dfrac{\%Succinoyl}{M(Succinoyl)}}{\dfrac{\%cellulose\ backbone}{M(AGU)}}$$

$M(MeO) = M(OCH_3) = 31.03$ Da $M(HPO) = M(OCH_2CH(OH)CH_3) = 75.09$ Da $M(Acetyl) = M(COCH_3) = 43.04$ Da $M(Succinoyl) = M(COC_2H_4COOH) = 101.08$ Da $M(AGU) = 162.14$ Da $M(OH) = 17.008$ Da $M(H) = 1.008$ Da

By convention, the weight percent is an average weight percentage based on the total weight of the cellulose repeat unit, including all substituents. The content of the methoxyl group is reported based on the mass of the methoxyl group (i.e., $-OCH_3$). The content of the hydroxyalkoxyl group is reported based on the mass of the hydroxyalkoxyl group (i.e., $-O$-alkylene$-OH$); such as hydroxypropoxyl (i.e., $-O-CH_2CH(CH_3)-OH$). The content of the aliphatic monovalent acyl groups is reported based on the mass of $-C(O)-R_1$ wherein $R_1$ is a monovalent aliphatic group, such as acetyl ($-C(O)-CH_3$). The content of the group of formula $-C(O) - R - COOH$ is reported based on the mass of this group, such as the mass of succinoyl groups (i.e., $-C(O)-CH_2-CH_2-COOH$).

The esterified cellulose ether generally has a viscosity of up to 200 mPa·s, preferably up to 100 mPa·s, more preferably up to 50 mPa·s, and most preferably up to 5.0 mPa·s, measured as a 2.0 wt.-% solution of the esterified cellulose ether in 0.43 wt.-% aqueous NaOH at 20 °C. Generally the viscosity is at least 1.2 mPa·s, more typically at least 1.8 mPa·s, even more typically at least 2.4 mPa·s, and most typically at least 2.8 mPa·s, measured as a 2.0 wt.-% solution of the esterified cellulose ether in 0.43 wt.-% aqueous NaOH at 20 °C. The 2.0 % by weight solution of the esterified cellulose ether is prepared as described in"Hypromellose Acetate Succinate, United States Pharmacopeia and National Formulary, NF 29, pp. 1548-1550", followed by an Ubbelohde viscosity measurement according to DIN 51562-1:1999-01 (January 1999).

The esterified cellulose ether generally has a weight average molecular weight $M_w$ of up to 500,000 Dalton, preferably up to 250,000 Dalton, and more preferably up to 150,000 Dalton. Generally it has a weight average molecular weight $M_w$ of at least 10,000 Dalton, preferably at 15,000 Dalton, and most preferably at least 30,000 Dalton.

In the process of the present invention for at least partially dissolving the esterified cellulose ether a) the esterified cellulose ether is mixed with an aqueous liquid, and b) the degree of neutralization of the groups $- C(O) - R - COOH$ of the esterified cellulose ether in the aqueous liquid is maintained at or adjusted to less than 0.45, generally not more than 0.4, preferably not more than 0.3 or 0.2 or 0.1, more preferably not more than 0.05 or 0.01, and most preferably not more than $10^{-3}$ or even not more than $10^{-4}$. The term "degree of neutralization" as used herein defines the ratio of deprotonated carboxylic groups over the sum of deprotonated and protonated carboxylic groups, i.e.,

Degree of neutralization = [-C(O)-R-COO$^-$] / [-C(O)-R-COO$^-$ + -C(O)-R-COOH].

Mixing of the esterified cellulose ether with the aqueous liquid and maintaining or adjusting the degree of neutralization of the groups $- C(O) - R - COOH$ of the esterified cellulose ether can be conducted simultaneously. For example, an esterified cellulose ether can be chosen wherein the degree of neutralization of the groups $- C(O) - R - COOH$ is less than 0.45. When the aqueous liquid does not contain a basic compound that increases the degree of neutralization of the groups - C(O) - R - COOH to 0.45 or more, the degree of neutralization will be less than 0.45 already during the mixing process.

Alternatively, the esterified cellulose ether can first be mixed with the aqueous liquid and the degree of neutralization of the groups - C(O) - R - COOH can subsequently be set to less than 0.45. For example, when the esterified cellulose ether has a degree of neutralization of the groups - C(O) - R - COOH of 0.45 or more and/or the aqueous liquid comprises a basic compound, the degree of neutralization of the groups - C(O) - R - COOH is controlled after mixing the esterified cellulose ether with the aqueous liquid and is lowered to less than 0.45 if needed. E.g., an acid can be added to set the degree of neutralization to less than 0.45. However, preferably the esterified cellulose ether and the aqueous liquid are chosen that no addition of an acid is needed.

The temperature of the aqueous liquid with which the esterified cellulose ether is mixed preferably is 0 °C or more, typically 0.5°C or more. The temperature of the aqueous liquid is typically up to 20 °C, preferably less than 10 °C, more preferably less than 8 °C, even more preferably less than 5 °C, and most preferably up to 3 °C. Generally the esterified cellulose ether is blended with at least 5 weight parts, preferably at least 10 weight parts, more preferably at least 20 weight parts, and generally up to 100 weight parts, preferably up to 60 weight parts, more preferably up to 40 weight parts, of aqueous liquid per weight part of esterified cellulose ether.

It is essential in the process of the present invention that the temperature of the resulting mixture of the esterified cellulose ether and the aqueous liquid is set to less than 10°C, preferably less than 8 °C, more preferably less than 5 °C, and most preferably to 3 °C or less. The temperature of the resulting mixture is generally set to at least minus 2 °C, typically to 0 °C or more, and more typically to 0.5°C or more. It is not essential whether the temperature of the aqueous liquid is adjusted before or after mixing with the esterified cellulose ether. Preferably the mixture is kept at the above-mentioned temperature for a time period of at least 10 minutes, preferably at least 30 minutes, and more preferably at least 2 hours. Depending on the type of esterified cellulose ether, the dissolution process in the aqueous liquid can take quite a long time. Generally the mixture of the esterified cellulose ether and the aqueous liquid is kept at the above-mentioned temperature for a time period of up to a week, preferably up to 72 hours, and more preferably up to 24 hours.

The aqueous liquid may additionally comprise a minor amount of an organic liquid diluent; however, the aqueous liquid should generally comprise at least 80, preferably at least 85, more preferably at least at least 90, and particularly at least 95 weight percent of water, based on the total weight of the aqueous liquid. The term "organic liquid diluent" as used herein means an organic solvent or a mixture of two or more organic solvents. Preferred organic liquid diluents are polar organic solvents having one or more heteroatoms, such as oxygen, nitrogen or halogen like chlorine. More preferred organic liquid diluents are alcohols, for example multifunctional alcohols, such as glycerol, or preferably monofunctional alcohols, such as methanol, ethanol, isopropanol or n-propanol; ethers, such as tetrahydrofuran, ketones, such as acetone, methyl ethyl ketone, or methyl isobutyl ketone; acetates, such as ethyl acetate; halogenated hydrocarbons, such as methylene chloride; or nitriles, such as acetonitrile. More preferably the organic liquid diluents have 1 to 6, most preferably 1 to 4 carbon atoms. The aqueous liquid may comprise a basic compound, but the degree of neutralization of the groups - C(O) - R - COOH of the esterified cellulose ether in the resulting blend of esterified cellulose ether and aqueous liquid should not be more than 0.4, preferably not more than 0.3 or 0.2 or 0.1, more preferably not more than 0.05 or 0.01, and most preferably not more than $10^{-3}$ or even not more than $10^{-4}$. Preferably the aqueous liquid does not comprise a substantial amount of a basic compound. More preferably, the aqueous liquid does not contain a basic compound. Most preferably, the aqueous liquid comprises from 80 to 100 percent, preferably 85 to 100 percent, more preferably 90 to 100 percent and most preferably 95 to 100 percent of water, and from 0 to 20 percent, preferably 0 to 15 percent, more preferably 0 to 10 percent, and most preferably 0 to 5 percent of an organic liquid diluent, based on the total weight of the aqueous liquid. Most preferably the aqueous liquid consists of water, e.g., deionized or distilled water.

Surprisingly, at least a portion of the above-described esterified cellulose ether comprising groups of the formula - C(O) - R - COOH can be dissolved in the aqueous liquid described above under the above-mentioned temperature conditions, i.e., at a temperature of less than 10°C, preferably less than 8 °C, more preferably less than 5 °C, and most preferably to 3 °C or less, even when the esterified cellulose ether in the aqueous liquid has a degree of neutralization of the groups - C(O) - R - COOH of less than 0.45 or a preferred range listed above, e.g., when the esterified cellulose ether is blended with only water, such as deionized or distilled water. After the partial dissolution of the esterified cellulose ether in the aqueous liquid, the temperature of the aqueous composition comprising dissolved esterified cellulose ether can be slightly increased, e.g., to a temperature of not more than 25 °C, typically not more than 20 °C, without participation of the dissolved esterified cellulose ether. Only upon further temperature increase, e.g., to 30 °C or more, dissolved esterified cellulose ether precipitates.

The concentration at which the esterified cellulose ether is soluble in the aqueous liquid depends to a large extent on the total degree of ester substitution and to some extent also on the weight average molecular weight of the esterified cellulose ether. When the total degree of ester substitution of the esterified cellulose ether is not more than 1.0, preferably not more than 0.90, and more preferably not more than 0.80, a substantial percentage of the esterified cellulose ether can be dissolved in the aqueous liquid in the process of the present invention. Such esterified cellulose ether typically has solubility properties that at least 3 wt.%, typically at least 5 wt.%, and in preferred embodiments at least 10 wt.%, of the esterified cellulose ether is soluble in a mixture of 2.5 weight parts of the esterified cellulose ether and 97.5 weight parts of water at 2 °C. Typically this degree of solubility is also observed in a mixture of 5 or 10 weight parts of the esterified cellulose ether and 95 or 90 weight parts of water at 2 °C or even in a mixture of 20 weight parts of the esterified cellulose ether and 80 weight parts of water at 2 °C.

When the total degree of ester substitution is not more than 0.70 or even not more than 0.65, the esterified cellulose ether is completely or nearly completely soluble in the aqueous liquid at a concentration of 2.5% when carrying out the process of the present invention. More specifically, such esterified cellulose ether typically has solubility properties that at least 80 wt.%, preferably at least 85 wt.%, more preferably at least 90 wt.%, and most preferably at least 95 wt.% of the esterified cellulose ether is soluble in a mixture of 2.5 weight parts of the esterified cellulose ether and 97.5 weight parts of water at 2 °C. Typically this degree of solubility is also observed in a mixture of 5 or 10 weight parts of the esterified cellulose ether and 95 or 90 weight parts of water at 2 °C or even in a mixture of 20 weight parts of the esterified cellulose ether and 80 weight parts of water at 2 °C.

When the total degree of ester substitution is not more than 0.60 or even not more than 0.50, the esterified cellulose ether is completely soluble in the aqueous liquid at a concentration of 2.5% when carrying out the process of the present invention.

Moreover, of a given esterified cellulose ether the polymer chains of lower molecular weight, e.g. those of up to 1500,000 Dalton or even up to 100,000 Dalton are better soluble in the aqueous liquid that the longer polymer chains. The water-soluble portion of the esterified cellulose ether generally has a weight average molecular weight of at least 8,000 Dalton, typically at least 10,000 Dalton, and more typically at least 11,000 Dalton or at least 12,000 Dalton. The water-soluble esterified cellulose ether preferably has a weight average molecular weight $M_w$ of up to 70,000 Dalton, more preferably up to 60,000 Dalton, and most preferably up to 50,000 Dalton or up to 40,000 Dalton.

The water-soluble portion of the esterified cellulose ether that is dissolved in the aqueous liquid according to the process of the present invention can be recovered from the aqueous liquid, e.g., by heating the aqueous liquid comprising the dissolved esterified cellulose ether to a temperature of at least 30 °C, preferably at least 45 °C, more preferably at least 60 °C, and most preferably at least 80 °C. Typically the aqueous liquid comprising the dissolved esterified cellulose ether is heated to a temperature of up to 98 °C, and more typically of up to 95 °C. At such temperatures the dissolved esterified cellulose ether precipitates. The precipitated esterified cellulose ether can be separated from the aqueous liquid in a known manner, such as by centrifugation or filtration or upon settling by decantation. The observed water-insolubility of this esterified cellulose ether upon heating is reversible. The separated esterified cellulose ether is soluble in an aqueous liquid at a temperature of less than 10 °C. Alternatively, the esterified cellulose ether that is dissolved in the aqueous liquid can be recovered by another known technique, such as freeze-drying or spray-drying. However, it is generally more preferred to utilize the aqueous liquid comprising dissolved esterified cellulose ether directly for manufacturing capsules, for coating dosage forms or for preparing solid dispersions of an active ingredient in an esterified cellulose ether instead of isolating the dissolved esterified cellulose ether before further use.

Another aspect of the present invention is an aqueous composition which comprises at least 1 weight percent of an esterified cellulose ether dissolved in an aqueous liquid, wherein i) the esterified cellulose ether comprises groups of the formula - C(O) - R - COOH having a degree of neutralization of less than 0.45, wherein R is a divalent group, and ii) the aqueous composition has a temperature of no more than 10 °C. Preferred esterified cellulose ethers, preferred degrees of neutralization and preferred aqueous liquids are described further above. The temperature of the aqueous composition is less than 10°C, preferably less than 8 °C, more preferably less than 5 °C, and most preferably 3 °C or less. The temperature of the aqueous composition is generally at least minus 2 °C, typically at least 0 °C, and more typically at least 0.5°C. The aqueous composition typically comprises at least 2 wt.-%, preferably at least 5 wt.-%, more preferably at least 10 wt.-%, and in some cases even at least 15 wt.-% esterified cellulose ether dissolved in the aqueous liquid. Aqueous compositions comprising up to 20 wt.-%, or in preferred embodiments even up to 30 wt.-%, of esterified cellulose ether dissolved in the aqueous liquid can generally be prepared at 2 °C. The term "x wt.-% esterified cellulose ether dissolved in the aqueous liquid at 2 °C" as used herein means that x g of the esterified cellulose ether is dissolved in (100 - x) g of the aqueous liquid, such as water, at 2 °C. The composition may comprise one or more active ingredients, most preferably one or more drugs. The term "drug" is conventional, denoting a compound having beneficial prophylactic and/or therapeutic properties when administered to an animal, especially humans. The aqueous composition may further comprise optional additives, such as coloring agents, pigments, opacifiers, flavor and taste improvers, antioxidants, and any combination thereof. Optional additives are preferably pharmaceutically acceptable.

Another aspect of the present invention is a process for manufacturing capsule shells wherein an aqueous composition comprising an esterified cellulose ether at least partially dissolved in an aqueous liquid is produced as described above and dipping pins are contacted with the aqueous composition or with the portion of the aqueous composition wherein esterified cellulose ether is dissolved. The dipping pins should have a higher temperature than the aqueous composition. Typically the aqueous composition having a temperature of 10 °C or less is contacted with dipping pins that have a temperature of at least 15 °C, preferably at least 20 °C, more preferably at least 30 °C, and up to 95 °C, preferably up to 70 °C, and more preferably up to 60 °C. The capsules have enteric properties. When the esterified cellulose ether is completely or nearly completely dissolved in the aqueous liquid according to the process of the present invention, the dipping pins are contacted with the aqueous composition which comprises the esterified cellulose ether dissolved in the aqueous liquid.

When the esterified cellulose ether is only partially dissolved in the aqueous liquid according to the process of the present invention, the dipping pins are contacted with the portion of the aqueous composition wherein esterified cellulose ether is dissolved. In one embodiment the non-dissolved portion of the esterified cellulose ether is separated from the portion of the aqueous composition wherein esterified cellulose ether is dissolved before the aqueous composition is contacted with the dipping pins. In a preferred embodiment the non-dissolved portion of the esterified cellulose ether is not separated but left as sediment in the aqueous composition. The dipping pins can simply be dipped into the supernatant liquid portion of the aqueous composition wherein esterified cellulose ether is dissolved. The procedure allows a very efficient process for producing enteric capsules from aqueous compositions wherein esterified cellulose ethers are only partially dissolved.

Another aspect of the present invention is a process for coating dosage forms, such as tablets, granules, pellets, caplets, lozenges, suppositories, pessaries or implantable dosage forms, wherein an aqueous composition comprising an esterified cellulose ether at least partially dissolved in an aqueous liquid is produced as described above and dosage forms are contacted with the aqueous composition or with the portion of the aqueous composition wherein esterified cellulose ether is dissolved. When the esterified cellulose ether is only partially dissolved in the aqueous liquid according to the process of the present invention, the dosage forms are contacted with the portion of the aqueous composition wherein esterified cellulose ether is dissolved, e.g., by spraying this portion of the aqueous composition onto the dosage forms.

Another aspect of the present invention is a process for preparing a solid dispersion of an active ingredient in an esterified cellulose ether wherein an aqueous composition comprising an esterified cellulose ether at least partially dissolved in an aqueous liquid is produced as described above and an active ingredient is dissolved in the aqueous composition or in the portion of the aqueous composition wherein esterified cellulose ether is dissolved, and the resulting aqueous composition, or the resulting portion of the aqueous composition wherein esterified cellulose ether and active ingredient are dissolved, is dried to produce the solid dispersion of an active ingredient in an esterified cellulose ether. A preferred drying method is by spray-drying. The term "spray-drying" refers to processes involving breaking up liquid mixtures into small droplets (atomization) and rapidly removing solvent from the mixture in a spray-drying apparatus where there is a strong driving force for evaporation of solvent from the droplets. Spray-drying processes and spray-drying equipment are described generally in Perry's Chemical Engineers' Handbook, pages 20-54 to 20-57 (Sixth Edition 1984). More details on spray-drying processes and equipment are reviewed by Marshall, "Atomization and Spray-Drying," 50 Chem. Eng. Prog. Monogr. Series 2 (1954), and Masters, Spray Drying Handbook (Fourth Edition 1985). A useful spray-drying process is described in the International Patent Application WO 2005/115330, page 34, line 7 - page 35, line 25.

Some embodiments of the invention will now be described in detail in the following Examples.

EXAMPLES

Unless otherwise mentioned, all parts and percentages are by weight. In the Examples the following test procedures are used.

Content of ether and ester groups

The content of ether groups in the esterified cellulose ether is determined in the same manner as described for "Hypromellose", United States Pharmacopeia and National Formulary, USP 35, pp 3467-3469.

The ester substitution with acetyl groups ($-CO-CH_3$) and the ester substitution with succinoyl groups ($-CO-CH_2-CH_2-COOH$) are determined according to Hypromellose Acetate Succinate, United States Pharmacopia and National Formulary, NF 29, pp. 1548-1550". Reported values for ester substitution are corrected for volatiles (determined as described in section "loss on drying" in the above HPMCAS monograph).

Viscosity of Hydroxypropyl Methyl Cellulose Acetate Succinate (HPMCAS)

The 10 wt.-% solution of HPMCAS in acetone was prepared by mixing 10.0 g HPMCAS, based on its dry weight, with 90.0 g of acetone under vigorous stirring at room temperature. The mixture was rolled on a roller mixer for about 24 hours. The solution was centrifuged at 2000 rpm for 3 minutes using a Megafuge 1.0 centrifuge, commercially available from Heraeus Holding GmbH, Germany. An Ubbelohde viscosity measurement according to DIN 51562-1:1999-01 (January 1999) was carried out. The measurement was done at 20 °C.

The 20 wt.-% solution of HPMCAS in acetone was prepared by mixing 20.0 g HPMCAS, based on its dry weight, with 80.0 g of acetone under vigorous stirring at room temperature. The mixture was rolled on a roller mixer for about 24 hours. The solution was centrifuged at 2000 rpm for 3 minutes using a Megafuge 1.0 centrifuge, commercially available from Heraeus Holding GmbH, Germany. An Ubbelohde viscosity measurement according to DIN 51562-1:1999-01 (January 1999) was carried out. The measurement was done at 20 °C.

Determination of $M_w$ and $M_n$

$M_w$ and $M_n$ are measured according to Journal of Pharmaceutical and Biomedical Analysis 56 (2011) 743 unless stated otherwise. The mobile phase was a mixture of 40 parts by volume of acetonitrile and 60 parts by volume of aqueous buffer containing 50 mM $NaH_2PO_4$ and 0.1 M $NaNO_3$. The mobile phase was adjusted to a pH of 8.0. Solutions of the cellulose ether esters were filtered into a HPLC vial through a syringe filter of 0.45 $\mu$m pore size. The exact details of measuring $M_w$ and $M_n$ are disclosed in the International Patent Application No. WO 2014/137777 in the section "Examples" under the title "Determination of $M_w$, $M_n$ and $M_z$". In all Examples the recovery rate was at least 95 %.

Water-Solubility

Qualitative determination: A 2 wt. percent mixture of HPMCAS and water was prepared by mixing 2.0 g HPMCAS, based on its dry weight, with 98.0 g water under vigorous stirring at 0.5°C for 16 hours. The temperature of the mixture of HPMCAS and water was then increased to 5 °C. The water solubility of the esterified cellulose ether was determined by visual inspection. The determination whether the HPMCAS was water-soluble at 2% at 5 °C or not was done as follows. "Water soluble at 2% - yes" means that a solution without sediment was obtained according to the procedure above. "Water soluble at 2% - no" means that at least a significant portion of the HPMCAS remained undissolved and formed sediment when mixing 2.0 g HPMCAS, based on its dry weight, with 98.0 g water according to the procedure above. "Water soluble at 2% - partially" means that only a small portion of the HPMCAS remained undissolved and formed sediment when mixing 2.0 g HPMCAS, based on its dry weight, with 98.0 g water according to the procedure above.

Quantitative determination: 2.5 weight parts of HPMCAS, based on its dry weight, were added to 97.5 weight parts of deionized water having a temperature of 2 °C followed by stirring for 6 hours at 2°C and storing for 16 h at 2°C. A weighed amount of this mixture was transferred to a weighed centrifuge vial; the transferred weight of the mixture was noted as M1 in g. The transferred weight of HPMCAS [M2] was calculated as (transferred weight of mixture in g/100 g * 2.5g). The mixture was centrifuged for 60 min at 5000 rpm (2823 xg, Biofuge Stratos centrifuge from Thermo Scientific) at 2 °C. After centrifugation an aliquot was removed from the liquid phase and transferred to a dried weighed vial. The weight of the transferred aliquot was recorded as M3 in g. The aliquot was dried at 105°C for 12 h. The remaining g of HPMCAS was weighted after drying and recorded as M4 in g.

The term "% water soluble at 2.5 %" in Table 2 below expresses the percentage of HPMCAS that is actually dissolved in the mixture of 2.5 weight parts of HPMCAS and 97.5 weight parts of deionized water. It is calculated as (M4 / M2) * (M1 / M3) * 100), which corresponds to (g HPMCAS in liquid aliquot / g HPMCAS transferred to centrifuge vial) * (g mixture transferred to centrifuge vial / g liquid aliquot after centrifugation).

Example 1

A hydroxypropyl methyl cellulose acetate succinate (HPMCAS-I) was used as a starting material for the dissolution trial. HPMCAS-I had been produced in a known manner by reacting a hydroxypropyl methylcellulose (HPMC) with acetic anhydride and succinic anhydride in the presence of glacial acetic acid and sodium acetate (water free). The HPMC contained 28.4 % methoxyl groups, 9.0 % hydroxypropoxyl groups and a viscosity of 3 mPa·s, measured as a 2 % solution in water at 20 °C according to ASTM D2363 - 79 (Reapproved 2006). The HPMC is commercially available from The Dow Chemical Company as Methocel E3 LV Premium cellulose ether. The produced HPMCAS-I was purified several times with water having a temperature of 23 °C. Totally 100 weight parts of water were used per 1 weight part of HPMCAS-I.

344 g of HPMCAS-I having a temperature of 20 °C was suspended in 2.83 liter of water having a temperature of 2°C under stirring for 2h and stored for 12h at 0.5 °C. The resulting mixture of HPMCAS-I and water had a temperature of 0.5 °C. A portion of the HPMCAS-I was dissolved in the mixture of HPMCAS-I and water, hereafter designated as "water-soluble portion".

Then the liquid portion of the mixture was separated from the suspended HPMCAS by centrifugation (Microfuge 1.0, Heraeus, 4000 rpm, 5min) at a temperature of 5 °C. The water-soluble portion of HPMCAS-I was precipitated from the liquid by heating the liquid to 95°C for 10 min. It was 14 % of the total amount of HPMCAS-I.

The properties of the starting material HPMCAS-I and of the water-soluble portion of HPMCAS-I are listed in Table 1 below.

Example 2

A HPMCAS-II was used as a starting material for the dissolution trial. HPMCAS-II had been produced in a known manner by reacting a hydroxypropyl methylcellulose (HPMC) with acetic anhydride and succinic anhydride in the presence of glacial acetic acid and sodium acetate (water free). The HPMC contained 28.7 % methoxyl groups, 9.0 % hydroxypropoxyl groups and a viscosity of 3 mPa·s, measured as a 2 % solution in water at 20 °C according to ASTM D2363 - 79 (Reapproved 2006). The HPMC is commercially available from The Dow Chemical Company as Methocel E3 LV Premium cellulose ether. The produced HPMCAS-II was purified several times with water having a temperature of 23 °C. Totally 100 weight parts of water were used per 1 weight part of HPMCAS-II. 100 g of HPMCAS-II having a temperature of 20°C was suspended in 5 liter of water having a temperature of 1.5 °C under stirring for 4 h. The resulting mixture of HPMCAS-II and water had a temperature of 2 °C. A portion of the HPMCAS-II was dissolved in the mixture of HPMCAS-II and water, hereafter designated as "water-soluble portion".

Then the liquid portion of the mixture was separated from the suspended HPMCAS by filtration over a metal sieve at a temperature of 5 °C. The water-soluble portion of HPMCAS-II was recovered as solid mass from the liquid by freeze-drying.

The properties of the starting material HPMCAS-II and of the water-soluble portion of HPMAS-II are listed in Table 1 below.

Example 3

A HPMCAS-III was used as a starting material for the dissolution trial. HPMCAS-III had been produced in a known manner by reacting a hydroxypropyl methylcellulose (HPMC) with acetic anhydride and succinic anhydride in the presence of glacial acetic acid and sodium acetate (water free). The same HPMC was used as in Example 2. 750g of HPMCAS-III having a temperature of 20 °C was suspended in 4.6 liter of water having a temperature of 2°C under stirring for 2h and stored for 12h at 3 °C. The resulting mixture of HPMCAS-III and water had a temperature of 3 °C. A portion of the HPMCAS-III was dissolved in the mixture of HPMCAS-III and water, hereafter designated as "water-soluble portion".

Then the liquid portion of the mixture was separated from the suspended HPMCAS by centrifugation (Microfuge 1.0, Heraeus, 10000 rpm, 20min) at a temperature of 1 °C. The water-soluble portion of HPMCAS-III was recovered as solid mass from the liquid by freeze-drying. 75 g of water-soluble HPMCAS was recovered (10 % of the total weight of HPMCAS-III).

The properties of the starting material HPMCAS-III and of the water-soluble portion of HPMAS-III are listed in Table 1 below.

Table 1

| Example | HPMCAS | Molecular weight (kDA) | | 10% viscosity in acetone [mPa·s] | 20% viscosity in acetone [mPa·s] | Ether Substitution | | Ester substitution | | Ether Substitution | | Ester substitution | |
|---|---|---|---|---|---|---|---|---|---|---|---|---|---|
| | | $M_w$ | $M_n$ | | | Methoxyl (%) | Hydroxypropoxyl (%) | Acetyl (%) | Succinoyl (%) | $DS_M$ | $MS_{HP}$ | $DOS_{Ac}$ | $DOS_S$ |
| 1 | HPMCAS-I starting material | 186 | 92 | 19.5 | n.a. | 23.4 | 7.5 | 11.8 | 7.3 | 1.88 | 0.25 | 0.69 | 0.18 |
| | HPMCAS-I water-soluble portion** | 63 | 29 | 6.4 | n.a. | 26.0 | 7.4 | 11.2 | 6.2 | 2.08 | 0.24 | 0.64 | 0.15 |
| 2 | HPMCAS-II starting material | 152 | 68 | 23 | n.a. | 23.2 | 7.3 | 9.7 | 11.2 | 1.91 | 0.25 | 0.57 | 0.28 |
| | HPMCAS-II water-soluble portion | 13 | 10 | n.a. | n.a. | 26.1 | 7.0 | 8.7 | 8.6 | 2.07 | 0.23 | 0.50 | 0.21 |
| 3 | HPMCAS-III starting material | 114 | 46 | 18 | n.a. | 22.8 | 7.2 | 8.0 | 14.5 | 1.92 | 0.25 | 0.49 | 0.38 |
| | HPMCAS-III water-soluble portion | 13.6 | 11.1 | 3.3 | 22.8 | 25.6 | 6.8 | 7.1 | 11.2 | 2.05 | 0.23 | 0.41 | 0.28 |
| ** average of 2 measurements   n.a.: not assessed | | | | | | | | | | | | | |

Example 4: Preparation of Capsules

HPMCAS-III was partially dissolved in water as described in Example 3 above. The water-soluble portion of HPMCAS-III was recovered as solid mass from the liquid by freeze-drying. The water-soluble, freeze-dried portion of HPMCAS-III was dissolved in deionized water at a temperature of 2°C and a concentration of 25 wt.-%.

Capsule shells were produced by dipping metallic pins having a temperature of 21 °C, 30 °C and 55 °C, respectively, into the HPMCAS solution having a temperature of 5°C. The pins were then withdrawn from the aqueous HPMCAS solution and a film was formed on the molding pins. Capsule shells formed on the pins at each of these temperatures. The capsule shells formed on pins having room temperature (21 °C) were dried at room temperature, the capsule shells formed on pins having a temperature of 30 °C were dried at 30 °C and the capsule shells formed on pins having a temperature of 55 °C were dried at 55 °C.

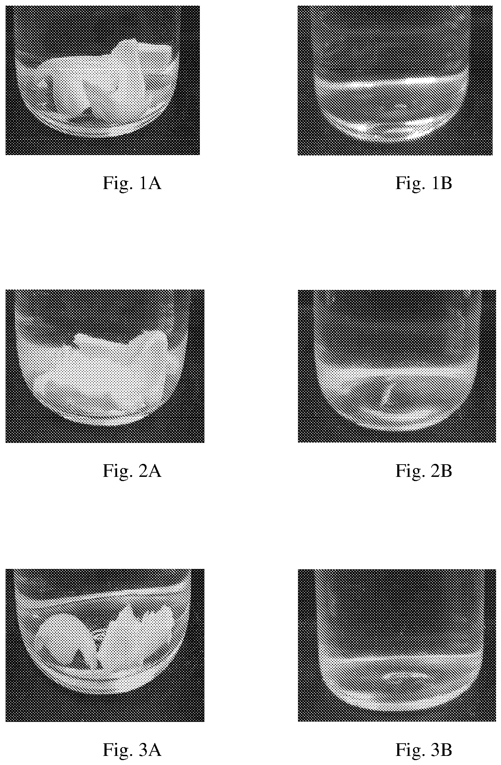

To test the solubility of the capsule shells in the acidic environment of the stomach, the capsule shells were broken into pieces and immersed into 0.1 N HCl. The capsule pieces were left there for 12 h at a temperature of 21°C. The capsule pieces did not dissolve in 0.1 N HCl during these 12 hours. The capsule pieces could be seen by the unprotected eye in 0.1 N HCl during these entire 12 hours. Fig. 1A, 2A and 3A show the non-dissolved pieces of capsule shells in 0.1 N HCl. Fig. 1A illustrates pieces of capsule shells prepared on pins having room temperature, Fig. 1B illustrates pieces of capsule shells prepared on pins having a temperature of 30 °C and Fig. 1C illustrates pieces of capsule shells prepared on pins having a temperature of 55 °C.

To test the solubility of the capsule shells in a neutral environment, the 0.1 N HCl was poured off from the capsule pieces and the capsule pieces were put into a McIlvaine's buffer solution (containing disodium monophosphate and citric acid) having a pH of 6.8. After about 60 minutes all pieces of capsule shells were completely dissolved in the buffer of pH 6.8 leaving clear solutions. Fig. 1B, 2B and 3B are photographical representations of the McIlvaine's buffer solution of pH 6.8 into which the non-dissolved pieces of capsule shells shown in Fig. 1A, 2A and 3A have been placed; all pieces of capsule shells are dissolved in the McIlvaine's buffer solution of pH 6.8.

Examples 3 and 4 illustrate that esterified cellulose ethers can be partially dissolved in water at a temperature of less than 10 °C, preferably less than 8 °C, more preferably less than 5 °C, and particularly at 3 °C or less. The water-soluble portion of the esterified cellulose ethers can be dissolved in water at a high concentration, e.g., at a concentration of 25 wt.-%.

The process of the present invention allows efficient and environmentally friendly production of capsule shells, coating of dosage forms or the production of solid dispersions of drugs in the esterified cellulose ethers at high throughput. Partial neutralization, which might impact the enteric properties of the esterified cellulose ethers, is not needed. Moreover, Example 4 illustrates that the capsules can be prepared even at room temperature.

Moreover, Table 1 above illustrates the low viscosity of the water-soluble portion of esterified cellulose ethers in acetone at 20 °C. The ability to provide highly concentrated solutions of the esterified cellulose ethers also in organic solvents such as acetone allows efficient processes for producing capsules or coatings from the water-soluble portion of the esterified cellulose ethers or for producing solid dispersions of drugs in the water-soluble portion of esterified cellulose ethers at high throughput from aqueous compositions, from compositions comprising one or more organic solvents or from liquid compositions which comprise a mixture of water and one or more organic solvents.

Example 5 - 24

HPMCAS was produced by esterifying HPMC with succinic anhydride and acetic anhydride. The HPMC had a methoxyl substitution ($DS_M$) and hydroxypropoxyl substitution ($MS_{HP}$) as listed in Table 2 below and a viscosity of 3.0 mPa·s measured as a 2 % solution in water at 20 °C according to ASTM D2363 - 79 (Reapproved 2006). The weight average molecular weight of the HPMC was about 20,000 Dalton. The HPMC is commercially available from The Dow Chemical Company as Methocel E3 LV Premium cellulose ether. HPMCAS samples having the properties listed in Table 2 below were produced.

A 2 wt. percent mixture of HPMCAS and water was prepared by mixing 2.0 g HPMCAS, based on its dry weight, with 98.0 g water under vigorous stirring at 0.5°C for 16 hours. The temperature of the mixture of HPMCAS and water was then increased to 5 °C. The esterified cellulose ethers of Examples 5 - 24 were dissolved at a concentration of 2 wt.-% in water at 5 °C. When the temperature of the prepared HPMCAS solution in water was increased to 20°C (room temperature), no precipitation occurred.

Table 2

| (Comparative) Ex. | Molecular weight (kDA) | | 10% viscosity in acetone [mPa·s] | Methoxyl (%) | Hydroxy - propoxyl (%) | Acety l (%) | Succinoyl (%) | $DS_M$ | $MS_{HP}$ | $DS_{Ac}$ | $DS_s$ | Sum $DS_{Ac}$ + $DS_s$ | % water soluble at 2.5 % | Water- soluble at 5 °C |
|---|---|---|---|---|---|---|---|---|---|---|---|---|---|---|
| | $M_n$ | Mw | | | | | | | | | | | | |
| 5 | 25 | 89 | 12.0 | 26.8 | 8.5 | 7.1 | 2.1 | 1.93 | 0.26 | 0.37 | 0.05 | 0.42 | 99 | yes* |
| 6 | 25 | 114 | 13.0 | 26.4 | 8.3 | 7.6 | 2.1 | 1.91 | 0.25 | 0.40 | 0.05 | 0.45 | 100 | yes |
| 7 | 17 | 39 | n.m. | 26.0 | 7.9 | 7.3 | 4.2 | 1.92 | 0.24 | 0.39 | 0.10 | 0.49 | 100 | yes |
| 8 | 18 | 29 | n.m. | 26.8 | 8.2 | 6.8 | 2.6 | 1.93 | 0.24 | 0.35 | 0.06 | 0.41 | 100 | yes* |
| 9 | 20 | 27 | n.m. | 27.4 | 8.3 | 5.3 | 3.5 | 1.97 | 0.25 | 0.28 | 0.08 | 0.36 | 99 | yes* |
| 10 | 20 | 27 | n.m. | 27.3 | 8.3 | 4.1 | 5.0 | 1.97 | 0.25 | 0.21 | 0.11 | 0.32 | 101 | yes* |
| 11 | 20 | 26 | n.m. | 27.8 | 8.3 | 2.4 | 5.9 | 1.99 | 0.25 | 0.12 | 0.13 | 0.25 | 101 | yes* |
| 12 | 18 | 26 | n.m. | 26.6 | 8.2 | 6.1 | 4.3 | 1.95 | 0.25 | 0.32 | 0.10 | 0.42 | 101 | yes* |
| 13 | 20 | 52 | n.m. | 25.6 | 7.9 | 6.8 | 6.6 | 1.93 | 0.25 | 0.37 | 0.15 | 0.52 | 101 | yes |
| 14 | 22 | 57 | n.m. | 25.1 | 7.9 | 5.3 | 8.3 | 1.90 | 0.25 | 0.29 | 0.19 | 0.48 | 100 | yes |
| 15 | 23 | 57 | n.m. | 24.8 | 7.7 | 3.2 | 11.4 | 1.89 | 0.24 | 0.18 | 0.27 | 0.45 | 100 | yes |
| 16 | 29 | 74 | n.m. | 24.3 | 7.7 | 7.8 | 7.2 | 1.86 | 0.24 | 0.43 | 0.17 | 0.60 | 100 | yes |
| 17 | 27 | 67 | n.m. | 24.2 | 7.7 | 8.0 | 7.5 | 1.86 | 0.24 | 0.44 | 0.18 | 0.62 | 96 | yes |
| 18 | 30 | 87 | n.m. | 24.2 | 7.7 | 8.3 | 7.6 | 1.87 | 0.25 | 0.46 | 0.18 | 0.64 | 95 | yes |
| 19 | 36 | 92 | n.m. | 23.1 | 7.8 | 9.0 | 7.6 | 1.80 | 0.25 | 0.50 | 0.18 | 0.68 | 79 | partially |
| 20 | 58 | 176 | n.m. | 24.2 | 7.7 | 7.3 | 8.4 | 1.87 | 0.25 | 0.41 | 0.20 | 0.61 | 81 | partially |
| 21 | 29 | 57 | n.m. | 23.8 | 7.7 | 9.4 | 5.9 | 1.82 | 0.24 | 0.52 | 0.14 | 0.66 | 99 | yes |
| 22 | 22 | 65 | 23 | 24.1 | 7.7 | 9.3 | 7.4 | 1.89 | 0.25 | 0.52 | 0.18 | 0.70 | 83 | partially |
| 23 | 23 | 60 | 122 | 24.4 | 7.7 | 8.4 | 6.6 | 1.87 | 0.24 | 0.46 | 0.16 | 0.62 | 85 | partially |
| 24 | 22 | 64 | 89 | 24.7 | 7.7 | 8.7 | 5.5 | 1.87 | 0.24 | 0.48 | 0.13 | 0.60 | 90 | yes |

n.m.: not measured *very clear solution

Examples 25 - 27

HPMCAS samples were used as starting materials that are known by the trade name "AQOAT". Shin-Etsu manufactures three grades of AQOAT polymers that have different combinations of substituent levels to provide enteric protection at various pH levels, AS-L, AS-M, and AS-H, typically followed by the designation "F" for fine or "G", such as AS-LF or AS-LG. Their sales specifications are listed in Table 1 on page 2 of WO 2011/159626 and in WO 2014/137777 on page 24. According to the Technical Brochure of Shin-Etsu "Shin-Etsu AQOAT Enteric Coating Agent" edition 04.9 05.2/500, all grades of AQOAT polymers are soluble in 10% NaOH but insoluble in purified water.

| Item | Substituent | L Grades | | M Grades | | H Grades | |
|---|---|---|---|---|---|---|---|
| | | Range* | Average (of 12 lots) | Range* | Average (of 28 lots) | Range* | Average (of 17 lots) |
| Manufacturer's Certificate of Analysis (wt%) | Methoxyl | 21.7-22.5 | 22.1 ± 0.3 | 22.7-23.6 | 23.1 ± 0.2 | 23.2-24.1 | 23.7 ± 0.3 |
| | Hydroxypropoxyl | 6.8-7.1 | 7.0±0.1 | 7.0-7.9 | 7.3 ± 0.2 | 7.1-7.8 | 7.5 ± 0.2 |
| | Acetyl | 7.2-8.1 | 7.7 ± 0.3 | 8.7-10.8 | 9.3 ± 0.4 | 11.0-12.2 | 11.5 ± 0.3 |
| | Succinoyl | 15.1-16.5 | 15.5 ± 0.4 | 10.8-11.5 | 11.2 ± 0.2 | 5.3-7.6 | 6.5 ± 0.7 |
| Calculated Degree of Substitution** | DOSM | 1.84-1.91 | 1.87 ± 0.03 | 1.85-1.94 | 1.89 ± 0.02 | 1.84-1.92 | 1.88 ± 0.02 |
| | DOSHP | 0.24-0.25 | 0.25 ± 0.01 | 0.24-0.27 | 0.25 ± 0.01 | 0.23-0.26 | 0.24 ± 0.01 |
| | DOSAc | 0.44-0.49 | 0.47 ± 0.02 | 0.51-0.65 | 0.55 ± 0.03 | 0.62-0.70 | 0.66 ± 0.02 |
| | DOSs | 0.39-0.43 | 0.40 ± 0.01 | 0.27-0.29 | 0.28 ± 0.01 | 0.13-0.19 | 0.16 ± 0.02 |
| | DOSM + DOSAc + DOSs | 2.70-2.80 | 2.75 ± 0.03 | 2.65-2.87 | 2.71 ± 0.03 | 2.63-2.73 | 2.70 ± 0.03 |
| | DOSAc+ DOSs | 0.85-0.89 | 0.88 ± 0.01 | 0.80-0.93 | 0.83 ± 0.03 | 0.77-0.84 | 0.81 ± 0.02 |
| * Range of several lots of polymer for each grade (the number of lots is indicated under "Average"). | | | | | | | |
| **Degree of substitution calculated as described in WO 2011/159626 | | | | | | | |

A quantitative determination of the water-solubility of the HPMCAS samples at 2 °C in a mixture of 2.5 weight parts of HPMCAS and 97.5 weight parts of deionized water was carried out as described under the paragraph "Water-Solubility" above. The percentage of HPMCAS that was actually dissolved in the mixture of 2.5 weight parts of HPMCAS and 97.5 weight parts of deionized water having a temperature of 2 °C is listed in Table 3 below.

Table 3

| Example | HPMCAS | % dissolved HPMCAS in mixture of 2.5 parts HPMCAS and 97.5 parts of water at 2 °C |
|---|---|---|
| 25 | L Grade starting material (Batch No. 0093229) | 12 |
| | water-soluble portion | 100 |
| 26 | M Grade starting material (Batch No. 0083210) | 46 |
| | M Grade water-soluble portion | 100 |

(continued)

| Example | HPMCAS | % dissolved HPMCAS in mixture of 2.5 parts HPMCAS and 97.5 parts of water at 2 °C |
|---|---|---|
| 27 | H Grade starting material (Batch No. 9053119) | 31 |
| | H Grade water-soluble portion | 100 |

When trying to dissolve the HPMCAS samples at 21 °C instead of at 2 °C, no significant portion of the HPMCAS samples could be dissolved. All HPMCAS samples were insoluble in water at 21 °C, as described the above-mentioned Technical Brochure of Shin-Etsu "Shin-Etsu AQOAT Enteric Coating Agent".

**Claims**

1. A process for producing an aqueous composition comprising at least 1 weight percent of an esterified cellulose ether dissolved in an aqueous liquid, wherein the esterified cellulose ether comprises groups of the formula - C(O) - R - COOH, wherein R is a divalent hydrocarbon group and the process comprises the step of

   a) mixing the esterified cellulose ether with the aqueous liquid, and
   b) maintaining or adjusting the degree of neutralization of the groups -C(O)-R-COOH of the esterified cellulose ether at or to less than 0.45 and setting the temperature of the mixture of the esterified cellulose ether and the aqueous liquid to less than 10 °C to at least partially dissolve the esterified cellulose ether in the aqueous liquid to provide the aqueous composition comprising at least 1 weight percent esterified cellulose ether dissolved in the aqueous liquid.

2. The process of claim 1 wherein the mixture of the esterified cellulose ether and the aqueous liquid is kept at a temperature of less than 10 °C for at least 1 hour.

3. The process of claim 1 or 2 wherein the temperature of the mixture of the esterified cellulose ether and the aqueous liquid is set to less than 5 °C.

4. The process of any one of claims 1 to 3 wherein after at least partial dissolution of the esterified cellulose ether in the aqueous liquid at a temperature of less than 10 °C the temperature of the aqueous composition is increased to not more than 25 °C.

5. The process of any one of claims 1 to 4 wherein the esterified cellulose ether comprises groups of the formula -C(O)-CH$_2$-CH$_2$-COOH and additionally comprises aliphatic monovalent acyl groups.

6. The process of any one of claims 1 to 5 wherein the esterified cellulose ether is hydroxypropyl methylcellulose acetate succinate.

7. The process of any one of claims 1 to 6 wherein the total degree of ester substitution of the esterified cellulose ether is up to 1.0.

8. The process of any one of claims 1 to 7 wherein the aqueous composition comprises at least 2 wt.-% esterified cellulose ether dissolved in the aqueous liquid.

9. A process for manufacturing capsule shells comprising the steps of

   - producing, according to the process of any one of claims 1 to 8, an aqueous composition comprising at least 1 weight percent of an esterified cellulose ether dissolved in an aqueous liquid, wherein the esterified cellulose ether comprises groups of the formula -C(O)-R-COOH, wherein R is a divalent hydrocarbon group and
   - contacting dipping pins having a higher temperature than the aqueous composition with the aqueous composition or with the portion of the aqueous composition wherein esterified cellulose ether is dissolved.

10. A process for coating dosage forms comprising the steps of

- producing, according to the process of any one of claims 1 to 8, an aqueous composition comprising at least 1 weight percent of an esterified cellulose ether dissolved in an aqueous liquid, wherein the esterified cellulose ether comprises groups of the formula -C(O)-R-COOH, wherein R is a divalent hydrocarbon group and
- contacting dosage forms with the aqueous composition or with the portion of the aqueous composition wherein esterified cellulose ether is dissolved.

11. A process for preparing a solid dispersion of an active ingredient in an esterified cellulose ether comprising the steps of

- producing, according to the process of any one of claims 1 to 8, an aqueous composition comprising at least 1 weight percent of an esterified cellulose ether dissolved in an aqueous liquid, wherein the esterified cellulose ether comprises groups of the formula -C(O)-R-COOH, wherein R is a divalent hydrocarbon group,
- dissolving an active ingredient in the aqueous composition or in the portion of the aqueous composition wherein esterified cellulose ether is dissolved, and
- drying the aqueous composition or the portion of the aqueous composition wherein esterified cellulose ether and active ingredient are dissolved to produce the solid dispersion of an active ingredient in an esterified cellulose ether.

12. An aqueous composition comprising at least 1 weight percent of an esterified cellulose ether dissolved in an aqueous liquid, wherein

i) the esterified cellulose ether comprises groups of the formula -C(O)-R-COOH having a degree of neutralization of less than 0.45, wherein R is a divalent hydrocarbon group, and
ii) the aqueous composition has a temperature of no more than 10 °C.

13. The aqueous composition of claim 12 wherein the esterified cellulose ether comprises groups of the formula -C(O)-CH2-CH$_2$-COOH and additionally comprises aliphatic monovalent acyl groups.

14. The aqueous composition of claim 12 or 13 wherein the total degree of ester substitution of the esterified cellulose ether is up to 1.0.

15. The aqueous composition of any one of claims 12 to 14 wherein the esterified cellulose ether is hydroxypropyl methylcellulose acetate succinate wherein the degree of neutralization of the groups -C(O)-CH$_2$-CH$_2$-COOH is not more than 0.1.


**Patentansprüche**

1. Verfahren zur Herstellung einer wässrigen Zusammensetzung, enthaltend wenigstens 1 Gew.-% eines veresterten Celluloseethers gelöst in einer wässrigen Flüssigkeit, wobei der veresterte Celluloseether Gruppen der Formel -C(O)-R-COOH enthält, worin R eine zweibindige Kohlenwasserstoffgruppe ist und das Verfahren die Schritte umfasst:

a) Mischen des veresterten Celluloseethers mit der wässrigen Flüssigkeit und
b) Aufrechterhalten oder Einstellung des Neutralisationsgrades der Gruppen -C(O)-R-COOH des veresterten Celluloseethers bei oder weniger als 0,45 und Festsetzen der Temperatur der Mischung des veresterten Celluloseethers und der wässrigen Flüssigkeit auf weniger als 10°C, um mindestens teilweise den veresterten Celluloseether in der wässrigen Flüssigkeit zu lösen, um die wässrige Zusammensetzung, enthaltend wenigstens 1 Gew.-% verestertem Celluloseether gelöst in der wässrigen Flüssigkeit, bereitzustellen.

2. Verfahren nach Anspruch 1, wobei die Mischung des veresterten Celluloseethers und der wässrigen Flüssigkeit bei einer Temperatur von weniger als 10°C wenigstens eine Stunde lang gehalten wird.

3. Verfahren nach Anspruch 1 oder 2, wobei die Temperatur der Mischung des veresterten Celluloseethers und der wässrigen Flüssigkeit auf weniger als 5°C festgelegt wird.

4. Verfahren nach einem der Ansprüche 1 bis 3, wobei nach mindestens teilweiser Auflösung des veresterten Celluloseethers in der wässrigen Flüssigkeit bei einer Temperatur von weniger als 10°C die Temperatur der wässrigen Zusammensetzung auf nicht mehr als 25°C erhöht wird.

5. Verfahren nach einem der Ansprüche 1 bis 4, wobei der veresterte Celluloseether Gruppen der Formel -C(O)-CH$_2$-CH$_2$-COOH und zusätzlich aliphatische monovalente Acylgruppen enthält.

6. Verfahren nach einem der Ansprüche 1 bis 5, wobei der veresterte Celluloseether Hydroxypropylmethylcelluloseacetatsuccinat ist.

7. Verfahren nach einem der Ansprüche 1 bis 6, wobei der Gesamtgrad der Estersubstitution des veresterten Celluloseethers bis zu 1,0 beträgt.

8. Verfahren nach einem der Ansprüche 1 bis 7, wobei die wässrige Zusammensetzung wenigstens 2 Gew.-% veresterten Celluloseether gelöst in der wässrigen Flüssigkeit enthält.

9. Verfahren zur Herstellung von Kapselschalen, umfassend die Schritte von:

   - Herstellen einer wässrigen Zusammensetzung, enthaltend wenigstens 1 Gew.-% eines veresterten Celluloseethers gelöst in einer wässrigen Flüssigkeit, wobei der veresterte Celluloseether Gruppen der Formel -C(O)-R-COOH enthält, worin R eine zweibindige Kohlenwasserstoffgruppe ist, gemäß dem Verfahren nach einem der Ansprüche 1 bis 8 und
   - In-Kontakt-Bringen von Tauchstiften, die eine höhere Temperatur als die der wässrigen Zusammensetzung aufweisen, mit der wässrigen Zusammensetzung oder mit dem Teil der wässrigen Zusammensetzung, in dem der veresterte Celluloseether gelöst ist.

10. Verfahren zur Beschichtung von Dosierformen, umfassend die Schritte von:

    - Herstellen einer wässrigen Zusammensetzung, enthaltend wenigstens 1 Gew.-% eines veresterten Celluloseethers, gelöst in einer wässrigen Flüssigkeit, wobei der veresterte Celluloseether Gruppen der Formel -C(O)-R-COOH enthält, worin R eine zweibindige Kohlenwasserstoffgruppe ist, gemäß dem Verfahren nach einem der Ansprüche 1 bis 8 und
    - In-Kontakt-Bringen der Dosierform mit der wässrigen Zusammensetzung oder mit dem Teil der wässrigen Zusammensetzung, in dem der veresterte Celluloseether gelöst ist.

11. Verfahren zur Herstellung einer festen Dispersion eines aktiven Bestandteils in einem veresterten Celluloseether, umfassend die Schritte von:

    - Herstellen einer wässrigen Zusammensetzung, enthaltend wenigstens 1 Gew.-% eines veresterten Celluloseethers gelöst in einer wässrigen Flüssigkeit, wobei der veresterte Celluloseether Gruppen der Formel -C(O)-R-COOH enthält, worin R eine zweibindige Kohlenwasserstoffgruppe ist, gemäß dem Verfahren nach einem der Ansprüche 1 bis 8 und
    - Auflösen eines aktiven Bestandteils in der wässrigen Zusammensetzung oder in dem Teil der wässrigen Zusammensetzung, in dem der veresterte Celluloseether gelöst ist, und
    - Trocknen der wässrigen Zusammensetzung oder des Teils der wässrigen Zusammensetzung, in dem der veresterte Celluloseether und der aktive Bestandteil gelöst sind, um die feste Dispersion eines aktiven Bestandteils in einem veresterten Celluloseether herzustellen.

12. Wässrige Zusammensetzung, enthaltend wenigstens 1 Gew.-% eines veresterten Celluloseethers gelöst in einer wässrigen Flüssigkeit, worin

    i) der veresterte Celluloseether Gruppen der Formel -C(O)-R-COOH mit einem Neutralisationsgrad von weniger als 0,45, worin R eine zweibindige Kohlenwasserstoffgruppe ist, enthält und
    ii) die wässrige Zusammensetzung eine Temperatur von nicht mehr als 10°C aufweist.

13. Wässrige Zusammensetzung nach Anspruch 12, wobei der veresterte Cellulsoeether Gruppen der Formel -C(O)-CH$_2$-CH$_2$-COOH und zusätzlich aliphatische monovalente Acylgruppen enthält.

14. Wässrige Zusammensetzung nach Anspruch 12 oder 13, wobei der Gesamtgrad an Estersubstitution des veresterten Celluloseether bis zu 1,0 ist.

15. Wässrige Zusammensetzung nach einem der Ansprüche 12 bis 14, wobei der veresterte Celluloseether Hydroxypropylmethylcelluloseacetatsuccinat ist und wobei der Neutralisationsgrad der Gruppen - C(O)-CH$_2$-CH$_2$-COOH nicht mehr als 0,1 beträgt.


**Revendications**

1. Un procédé pour la production d'une composition aqueuse comprenant au moins 1 pour cent en poids d'un éther de cellulose estérifié dissous dans un liquide aqueux, dans lequel l'éther de cellulose estérifié comprend des groupes de la formule -C(O)-R-COOH, dans laquelle R est un groupe hydrocarboné divalent et le procédé comprend l'étape consistant à

    a) mélanger l'éther de cellulose estérifié avec le liquide aqueux, et
    b) maintenir ou ajuster le degré de neutralisation des groupes - C(O) - R - COOH de l'éther de cellulose estérifié à 0,45 ou moins et régler la température du mélange de l'éther de cellulose estérifié et du liquide aqueux à moins de 10 °C afin de dissoudre au moins partiellement l'éther de cellulose estérifié dans le liquide aqueux afin d'obtenir la composition aqueuse comprenant au moins 1 pour cent en poids d'éther de cellulose estérifié dissous dans le liquide aqueux.

2. Le procédé de la revendication 1 dans lequel le mélange de l'éther de cellulose estérifié et du liquide aqueux est gardé à une température de moins de 10 °C pendant au moins 1 heure.

3. Le procédé de la revendication 1 ou de la revendication 2 dans lequel la température du mélange de l'éther de cellulose estérifié et du liquide aqueux est réglée à moins de 5 °C.

4. Le procédé de l'une quelconque des revendications 1 à 3 dans lequel après la dissolution au moins partielle de l'éther de cellulose estérifié dans le liquide aqueux à une température de moins de 10 °C, la température de la composition aqueuse est augmentée jusqu'à 25 °C au plus.

5. Le procédé de l'une quelconque des revendications 1 à 4 dans lequel l'éther de cellulose estérifié comprend des groupes de la formule -C(O)-CH$_2$-CH$_2$-COOH et comprend en plus des groupes acyle monovalents aliphatiques.

6. Le procédé de l'une quelconque des revendications 1 à 5 dans lequel l'éther de cellulose estérifié est l'acétate-succinate d'hydroxypropylméthylcellulose.

7. Le procédé de l'une quelconque des revendications 1 à 6 dans lequel le degré total de substitution ester de l'éther de cellulose estérifié va jusqu'à 1,0.

8. Le procédé de l'une quelconque des revendications 1 à 7 dans lequel la composition aqueuse comprend au moins 2 % en poids d'éther de cellulose estérifié dissous dans le liquide aqueux.

9. Un procédé pour la production d'enveloppes de capsule comprenant les étapes consistant à

    - produire, selon le procédé de l'une quelconque des revendications 1 à 8, une composition aqueuse comprenant au moins 1 pour cent en poids d'un éther de cellulose estérifié dissous dans un liquide aqueux, dans lequel l'éther de cellulose estérifié comprend des groupes de la formule - C(O) - R - COOH, dans laquelle R est un groupe hydrocarboné divalent et
    - mettre en contact des tiges d'immersion ayant une température plus élevée que la composition aqueuse avec la composition aqueuse ou avec la portion de la composition aqueuse dans laquelle de l'éther de cellulose estérifié est dissous.

10. Un procédé pour le revêtement de formes galéniques comprenant les étapes consistant à

    - produire, selon le procédé de l'une quelconque des revendications 1 à 8, une composition aqueuse comprenant au moins 1 pour cent en poids d'un éther de cellulose estérifié dissous dans un liquide aqueux, dans lequel l'éther de cellulose estérifié comprend des groupes de la formule - C(O) - R - COOH, dans laquelle R est un groupe hydrocarboné divalent et
- mettre en contact des formes galéniques avec la composition aqueuse ou avec la portion de la composition aqueuse dans laquelle de l'éther de cellulose estérifié est dissous.

11. Un procédé pour la préparation d'une dispersion solide d'un principe actif dans un éther de cellulose estérifié comprenant les étapes consistant à

- produire, selon le procédé de l'une quelconque des revendications 1 à 8, une composition aqueuse comprenant au moins 1 pour cent en poids d'un éther de cellulose estérifié dissous dans un liquide aqueux, dans lequel l'éther de cellulose estérifié comprend des groupes de la formule - C(O) - R - COOH, dans laquelle R est un groupe hydrocarboné divalent,
- dissoudre un principe actif dans la composition aqueuse ou dans la portion de la composition aqueuse dans laquelle de l'éther de cellulose estérifié est dissous, et
- faire sécher la composition aqueuse ou la portion de la composition aqueuse dans laquelle de l'éther de cellulose estérifié et un principe actif sont dissous afin de produire la dispersion solide d'un principe actif dans un éther de cellulose estérifié.

12. Une composition aqueuse comprenant au moins 1 pour cent en poids d'un éther de cellulose estérifié dissous dans un liquide aqueux, dans lequel

i) l'éther de cellulose estérifié comprend des groupes de la formule - C(O) - R - COOH ayant un degré de neutralisation de moins de 0,45, dans laquelle R est un groupe hydrocarboné divalent, et
ii) la composition aqueuse a une température de 10 °C au plus.

13. La composition aqueuse de la revendication 12 dans laquelle l'éther de cellulose estérifié comprend des groupes de la formule -C(O)-CH$_2$-CH$_2$-COOH et comprend en plus des groupes acyle monovalents aliphatiques.

14. La composition aqueuse de la revendication 12 ou de la revendication 13 dans laquelle le degré total de substitution ester de l'éther de cellulose estérifié va jusqu'à 1,0.

15. La composition aqueuse de l'une quelconque des revendications 12 à 14 dans laquelle l'éther de cellulose estérifié est l'acétate-succinate d'hydroxypropylméthylcellulose dans laquelle le degré de neutralisation des groupes -C(O)-CH$_2$-CH$_2$-COOH est de 0,1 au plus.

Fig. 1A

Fig. 1B

Fig. 2A

Fig. 2B

Fig. 3A

Fig. 3B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4226981 A
- WO 2005115330 A
- US 4365060 A
- WO 2013164121 A
- WO 2014137777 A
- WO 2011159626 A

**Non-patent literature cited in the description**

- **MCGINITY ; JAMES W.** Aqueous Polymeric Coatings for Pharmaceutical Dosage Forms. M. Dekker, 1989, 105-113
- Hypromellose Acetate Succinate. United States Pharmacopia and National Formulary, NF. vol. 29, 1548-1550
- Hypromellose. United States Pharmacopeia and National Formulary. vol. 35, 3467-3469
- Hypromellose Acetate Succinate. United States Pharmacopeia and National Formulary, NF. vol. 29, 1548-1550
- Perry's Chemical Engineers' Handbook. 1984, 20-54, 20-57
- **MARSHALL.** Atomization and Spray-Drying. *50 Chem. Eng. Prog. Monogr. Series,* 1954, vol. 2
- **MASTERS.** Spray Drying Handbook. 1985
- Hypromellose Acetate Succinate. United States Pharmacopia and National Formulary. vol. 29, 1548-1550
- Shin-Etsu AQOAT Enteric Coating Agent. Technical Brochure of Shin-Etsu